# EUROPÄISCHE PATENTSCHRIFT

(11) **EP 1 881 783 B1**
(45) Veröffentlichungstag und Bekanntmachung des Hinweises auf die Patenterteilung: **12.01.2011**
(21) Anmeldenummer: 06723331.2
(22) Anmeldetag: 10.03.2006
(51) Int. Cl.: A61B 5/00, H04L 29/06

(54) **BLUTDRUCKMESSGERÄT**
SPHYGMOMANOMETER
TENSIOMETRE

(30) Priorität: 28.04.2005 DE 102005019751
(43) Veröffentlichungstag der Anmeldung: 30.01.2008
(73) Patentinhaber: KAZ Europe SA, 1003 Lausanne (CH)
(72) Erfinder: HOLLINGER, Stefan, 61476 Kronberg (DE); WUNDER, Dieter, 63679 Schotten (DE); HECK, Ulrich, 47800 Krefeld (DE); FREUND, Dirk, 65779 Kelkheim (DE); HARTTMANN, Brigitte, 65527 Niedernhausen (DE); RÖNNEBERG, Gerrit, 64289 Darmstadt (DE); SCHNAK, Fred, 61476 Kronberg (DE)
(74) Vertreter: Grünecker, Kinkeldey, Stockmair & Schwanhäusser Anwaltssozietät
(86) Internationale Anmeldenummer: PCT/EP2006/002201
(87) Internationale Veröffentlichungsnummer: WO 2006/114155

(56) Entgegenhaltungen:
- EP-A- 1 168 770
- DE-A1- 10 237 692
- US-A1- 2004 039 254
- US-A1- 2004 162 466
- US-A1- 2004 215 958

## Beschreibung

Die vorliegende Erfindung betrifft ein Blutdruckmessgerät mit den Merkmalen des Oberbegriffs des Anspruchs 1 mit einer Messvorrichtung zur Bestimmung von Blutdruckdaten sowie einer Datenübertragungseinheit mit einer externen Übertragungsschnittstelle zur Übertragung der Blutdruckdaten an externe Datenverarbeitungsgeräte und/oder zur Übertragung von Steuerungsdaten von externen Datenverarbeitungsgeräten an die Messvorrichtung.

Blutdruckmessgeräte werden zunehmend mit den Forderungen nach erweitertem Datenmanagement konfrontiert. Ein solches Datenmanagement kann sowohl dem Privatgebrauch dienen als auch Teil einer erweiterten Diagnose bzw. Therapie mit ärztlicher Kontrolle sein. Hierzu ist es bekannt, an Blutdruckmessgeräten Datenübertragungsschniftstellen vorzusehen, über die die in dem Blutdruckmessgerät gespeicherten Messdaten ausgelesen werden können. In der Praxis ergeben sich hierbei jedoch dadurch Probleme, dass die vorhandene Infrastruktur bei Ärzten und Krankenhäusern zu wenig universell ist, um sich breit zu etablieren. So gibt es Blutdruckmessgeräte, die Infrarotschnittstellen besitzen oder per Faxmodem Daten an Ärzte übertragen können. Dies erfordert jedoch, dass der jeweilige Arzt mit dem jeweiligen Blutdruckmessgerät kompatible Datenverarbeitungsgeräte besitzt. Zur Auswertung der in einem Blutdruckmessgerät ermittelten bzw. gespeicherten Messdaten wurde auch bereits vorgeschlagen, in eine USB-Schnittstelle am Blutdruckmessgerät ein Datenübertragungskabel einzustecken, um die Daten auf einen PC zu überspielen. Da jedoch an die Praxisausrüstung eines Arztes immer wieder spezielle Anforderungen gestellt werden, ist davon auszugehen, dass bei fehlender Standardisierung keine Festlegung auf ein spezielles Übertragungskonzept erfolgen kann.

DE 102 37692 A1 offenbart ein modulares Universaladapter-Telemedizinsystem. US 2004/016 2466 A1 offenbart ein drahtloses Gesundheitsüberwachungsssystemm.

Der vorliegenden Erfindung liegt daher die Aufgabe zugrunde, ein verbessertes Blutdruckmessgerät sowie eine verbesserte Datenübertragungseinheit für ein solches Blutdruckmessgerät zu schaffen, die die Datenübertragung vereinfachen und die Kompatibilität zwischen dem Blutdruckmessgerät und den externen Datenverarbeitungsanlagen in Arztpraxen, Krankenhäusern etc. verbessern.

Diese Aufgabe wird erfindungsgemäß durch ein Blutdruckmessgerät gemäß Patentanspruch 1 gelöst. Bevorzugte Ausgestaltungen der Erfindung sind Gegenstand der abhängigen Ansprüche.

Der Erfindung liegt der Gedanke zugrunde, das Blutdruckmessgerät und die Datenübertragungseinheit mit ihrer externen Übertragungsschnittstelle voneinander zu trennen und über eine Anschlussschnittstelle miteinander verbindbar zu machen, so dass das Blutdruckmessgerät in der praktischen Anwendung nicht starr an eine technische Datenübertragungsform gekoppelt ist, sondern vielmehr an die Erfordernisse seitens der ärztliche Infrastruktur angepasst werden kann. Erfindungsgemäß besitzt die Datenübertragungseinheit einen separaten austauschbaren Schnittstellenwandler, der lösbar mit einem Gerätekorpus des Blutdruckmessgeräts verbindbar und dabei mit einer internen Anschlussschnittstelle an eine Anschlussschnittstelle der Messvorrichtung anschließbar ist, und der zwischen seiner internen Anschlussschnittstelle und seiner externen Übertragungsschnittstelle ein erstes Daten-und/oder Übertragungsformat passend für die Messvorrichtung in ein zweites Daten-und/oder Übertragungsformat passend für das jeweilige externe Datenverarbeitungsgerät und/oder umgekehrt wandelt. Das Blutdruckmessgerät wird sozusagen mit einem Schnittstellenadapter versehen, der leicht austauschbar ist, so dass das Blutdruckmessgerät bezüglich seiner Datenübertragungsschnittstelle einfach an die hardwaretechnischen Gegebenheiten auf Seiten des Arztes oder Krankenhauses und insbesondere an länderspezifische Datenübertragungsmodi anpassbar ist.

Mit dem austauschbaren Schnittstellenwandler kann das Blutdruckmessgerät insbesondere sehr rasch an verschiedene internationale Länderstandards angepasst werden, obwohl das Blutdruckmessgerät in derselben Grundausstattung bzw. dasselbe Grundgerät weltweit verwendet werden kann. Durch Aufstecken bzw. Anbauen des passenden Schnittstellenwandlers wird das Grundgerät an den jeweils notwendigen Übertragungsstandards angepasst. Dabei stellt der Schnittstellenwandler nicht nur die geometrisch passende externe Übertragungsschnittstelle zur Verfügung, sondern transformiert das in dem Blutdruckmessgerät, genauer gesagt an dessen fest installierter Anschlussschnittstelle verwendete Datenformat und/oder zugehörige Übertragungsprotokoll in das jeweils benötigte Datenformat und Übertragungsprotokoll, welches von der an dem jeweiligen Datenverarbeitungsgerät vorhandene Schnittstelle vorgegeben wird. Es versteht sich, dass auch der Datensignalpegel als solcher von dem Schnittstellenadapter in geeigneter Weise angepasst werden kann. Der Schnittstellenwandler ist vorteilhafterweise bidirektional arbeitend ausgebildet, d. h. er wandelt das erste Daten- und/oder Übertragungsformat passend für die Messvorrichtung des Blutdruckmessgerätes in das zweite Daten- und/oder Übertragungsformat passend für die jeweilige externe Datenverarbeitungsanlage und umgekehrt um.

Es versteht sich, dass das Blutdruckmessgerät auch ohne den genannten Schnittstellenwandler betrieben werden kann, nämlich insbesondere dann, wenn das von der fest eingebauten Anschlussschnittstelle des Blutdruckmessgerätes bereitgestellte Daten- und Übertragungsformat bereits dann für das jeweilige externe Datenverarbeitungsgerät passend ist. In diesem Fall kann das Blutdruckmessgerät unmittelbar mit dem externen Datenverarbeitungsgerät beispielsweise über ein Kabel verbunden werden, um die Daten von dem Blutdruckmessgerät auszulesen bzw. Steuerungsdaten in dieses einzuspielen.

Die Anschlussschnittstelle des Blutdruckmessgerätes, an die der Schnittstellenwandler anschließbar ist, kann grundsätzlich verschieden ausgebildet sein. In einem Beispiel, welches das Verständnis der Erfindung erleichtert, kann die Anschlussschnittstelle als RS-232-Port ausgebildet sein. Ein solcher RS-232-Port, der an sich bei Personal Computern bekannt ist, ist eine universelle und leistungsfähige Schnittstelle, die es ermöglicht, extern mittels der separaten Datenübertragungseinheit eine Vielzahl von Formen von Datenübertragungsschnittstellen anzuschließen. Bei dieser Ausführung ist die interne Anschlussschnittstelle des Schnittstellenwandlers ebenfalls ein RS-232-Port, um an die entsprechende fest installierte Anschlussschnittstelle des Blutdruckmessgerätes angeschlossen werden zu können. In diesem Fall wandelt der Schnittstellenwandler das für den RS-232-Port spezifische Daten- und Übertragungsformat in das jeweils benötigte Daten- und Übertragungsformat für die externe Übertragungsschnittstelle.

In Weiterbildung der Erfindung kann die separate Datenübertra- f gungseinheit als externe Übertragungsschnittstelle eine Infrarotschnittstelle, ein Faxmodem, ein Chipkartenterminal, eine Kabelanschlussschnittstelle und/oder eine Blue Tooth-Einheit besitzen. Weitere Ausführungsformen der externen Übertragungsschnittstelle der Datenübertragungseinheit, wie z. B. eine USB-Schnittstelle, einen Firewire-Port, eine WLAN-Schnittstelle sind möglich. Hierdurch kann durch einfaches Austauschen der Datenübertragungseinheit das Blutdruckmessgerät an nahezu jede externe Datenverarbeitungsanlage angeschlossen werden, um Daten aus dem Blutdruckmessgerät auszulesen und in der Datenverarbeitungsanlage weiterzuverarbeiten und/oder Daten von der externen Datenverarbeitungsanlage in das Blutdruckmessgerät einzuspielen.

Der Schnittstellenwandler umfasst in Weiterbildung der Erfindung insbesondere einen Protokollwandler, der das an der internen Anschlussschnittelle vorgesehene Datenübertragungsprotokoll mit dem hiervon verschiedenen, an der externen Übertragungsschnittstelle benötigten Datenübertragungsprotokoll synchronisiert. Zudem werden mittels eines Formatkonvertierers weitere Datenformatmerkmale in der notwendigen Weise konvertiert, um die Datenübertragung sicherzustellen. Falls notwendig, ist weiterhin ein Pegelwandler in den Schnittstellenwandler integriert, um den Datensignalpegel von dem Messgeräte internen Pegel auf den am Datenverarbeitungsgerät benötigen Pegel zu heben bzw. zu senken. Wird beispielsweise in der zuvor beschriebenen Weise an dem Blutdruckmessgerät selbst ein fest installierter RS-232-Port und an der Datenübertragungseinheit als externe Übertragungsschnittstelle eine Blue Tooth-Einheit verwendet, wandelt der Schnittstellenwandler das RS-232-Port spezifische Datenformat und Übertragungsprotokoll in das Datenformat und Übertragungsprotokoll, wie es die Blue Tooth-Spezifikationen festschreiben.

Gemäß Anspruch 1 ist bei der Erfindung vorgesehen, dass die Anschlussschnittstelle des Blutdruckmessgerätes, mit der die externe Datenübertragungseinheit verbindbar ist, eine Chipkartenkontaktiereinheit bildet bzw. von dieser gebildet wird. Die externe Datenübertragungseinheit ist bei dieser Ausführung mit einem entsprechenden Anschlussadapter versehen, der in die Chipkartenkontaktiereinheit eingesetzt werden kann. Zwar ist das Blutdruckmessgerät bei dieser Ausführung an sich auf das Transfermedium der Chipkarte festgelegt, trotzdem ist es jedoch durch Einsetzen der externen Übertragungseinheit mit ihrem Anschlussadapter und dem Datenformatkonvertierer offen für alle Erweiterungen. Zweckmäßigerweise besitzt das Blutdruckmessgerät bei dieser Ausführung Unterscheidungsmittel zur Unterscheidung zwischen Chipkartenbetrieb und Schnittstellenbetrieb, d.h. das Blutdruckmessgerät kann erkennen, ob in die Chipkartenkontaktiereinheit eine Chipkarte eingesetzt ist, die mit Daten beschrieben werden kann, oder ob die Übertragungseinheit mit ihrem Anschlussadapter eingesetzt ist.

Vorteilhafterweise wird auf umständliche Kabelverbindungen zwischen dem Gerätekorpus des Blutdruckmessgerätes und der Übertragungseinheit verzichtet. Die Datenübertragungseinheit ist in Weiterbildung der Erfindung als Aufsatz ausgebildet, der über eine Steckverbindung mit dem Gerätekorpus des Blutdruckmessgerätes verbindbar ist

Regelmäßig ist es ausreichend, wenn die Datenübertragungseinheit, ihre externe Übertragungsschnittstelle und/oder die Anschlussschnirtstelle des Blutdruckmessgerätes unidirektional ausgebildet sind, insbesondere das Auslesen von im Blutdruckmessgerät erzeugten Messdaten erlauben.

In Weiterbildung der Erfindung kann die Datenübertragungseinheit und/oder die messgeräteinteme Anschlussschnittstelle bidirektional ausgebildet sein. Dies erlaubt zum einen die vorgenannte Datenübertragung vom Blutdruckmessgerät zu externen Datenverarbeitungsanlagen. Andererseits ist es auch möglich, Daten, insbesondere Steuerungsdaten von externen Datenverarbeitungsanlagen, in das Blutdruckmessgerät einzuspielen. Beispielsweise können hierdurch bestimmte Geräteeinstellungen wie Messzeitpunkte, Erinnerungsalarme etc. von außen in das Blutdruckmessgerät programmiert werden.

Ein solcher bidlrektionaler Schnittstellenbetrieb kann nach einem weiteren Aspekt der vorliegenden Erfindung auch dadurch erfolgen, dass anstelle der vorgenannten Datenübertragungseinheit mit externer Übertragungsschnittstelle unmittelbar ein externes Datenverarbeltungsgerät, insbesondere ein Computer in Form eines PCs oder Laptops, ein Palmtop oder dergleichen, unmittelbar über die Anschlussschnittstelle mit der Messvorrichtung des Blutdruckmessgerätes verbunden wird. Hierbei kann der Schnittstettenbetrieb weit Ober ein reines Auslesen bzw. Einspielen von Daten hinausgehen. Werden solche externen Peripheriegeräte mit erhöhter Rechenleistung und hochrangigen Display-Möglichkeiten angeschlossen, bildet das Blutdruckmessgerät lediglich noch ein Aufnahmemodul für die Messdaten. Der Ablauf der Messung kann vom externen Datenverarbeitungsgerät über Schnittstellenbefehle gesteuert werden. Gleichfalls findet die Auswertung der Messdaten in dem externen Gerät statt und unterliegt keinen funktionalen Beschränkungen, wie sie in einem typischen Blutdruckmessgerät zu finden sind. Hierdurch kann eine erhebliche Ausweitung des Leistungsspektrums erzielt werden.

Alternativ oder zusätzlich kann über die Schnittstelle des Blutdruckmessgerätes, insbesondere unmittelbar über dessen Anschlussschnittstelle, eine biometrische Erkennungseinrichtung an das Blutdruckmessgerät angeschlossen werden, die beispielsweise mittels eines Sensors zur Aufnahme des Fingerabdrucks eine Benutzeridentifikation ausführt, sodann Steuerungsdaten für das Blutdruckmessgerät in Abhängigkeit der Benutzeridentifikation bestimmt und diese sodann in das Blutdruckmessgerät einspielt, z. B. um Eigenschaften des Messalgorithmus auf den jeweiligen Benutzer abzustimmen.

Nach einer besonders vorteilhaften Ausführung der Erfindung kann der externe Datenübertragungsbaustein mit dem Schnittstellenwandler gleichzeitig einen Akkulader zur elektrischen Ladung eines im Gerätekorpus des Blutdruckmessgerätes vorgesehenen Akkus bilden. Der externe, austauschbare Baustein mit dem Schnittstellenwandler erfüllt hier jedoch eine Doppelfunktion. Einerseits kann hierdurch der Datenübertragungsstandart des Blutdruckmessgerätes an die Erfordernisse angepasst werden. Zugleich kann der Akku des Blutdruckmessgerätes aufgeladen werden. Auch hier ist es besonders vorteilhaft, dass der Akkulader von einem externen, austauschbarem Baustein gebildet wird, da hierdurch auch die elektrische Schnittstelle des Akkuladers an die jeweiligen Landesstandards angepasst werden können. Der Akkulader kann einen Stromanschluss entsprechend den jeweiligen Landesnormen aufweisen und zudem einen Pegelwandler besitzen, um beispielsweise die in Europa üblichen 230 V Netzspannung auf die Ladespannung für den geräteinternen Akku herunterzuregeln. Soll das Gerät für die USA adaptiert werden, wird der den Baustein enthaltende Akkulader ersetzt, um für die in den USA üblichen 110 V Netzspannung die benötigte Akkuladespannung herabzuführen.

Diese und weitere Merkmale gehen außer aus den Ansprüchen auch aus der nachfolgenden Beschreibung hervor, wobei die Merkmale einzeln oder in Kombination bzw. Unterkombination miteinander bei einer Ausführungsform der Erfindung oder auf anderen Gebieten verwirklicht sein können. Ein bevorzugtes Ausführungsbeispiel der Erfindung wird nachfolgend anhand der zugehörigen Zeichnungen erläutert. In diesen zeigen:
- Fig. 1:: eine schematische Darstellung eines Blutdruckmessgeräts nach einer bevorzug- ten Ausführung der Erfindung, bei dem eine separate Datenübertragungseinheit mit externer Übertragungsschnittstelle lösbar am Gerätekorpus des Blutdruck- messgerätes befestigbar ist, und
- Fig. 2:: eine schematische Darstellung des Blutdruckmessgeräts aus Fig. 1, bei dem anstelle der separaten Datenübertragungseinheit unmittelbar ein externes Da- tenverarbeitungsgerät in Form eines Laptops angeschlossen ist.

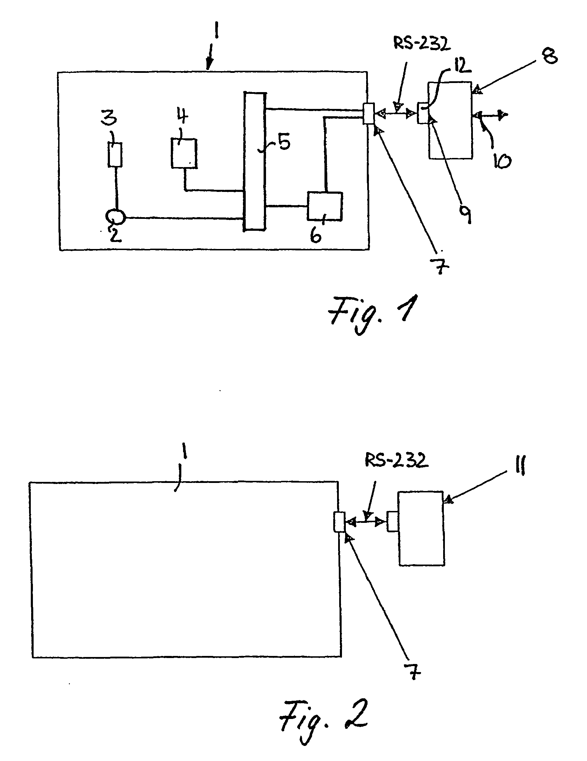

In an sich bekannter Weise kann das Blutdruckmessgerät gemäß Fig. 1, ohne eigens dargestellt zu sein, eine um ein Körperglied legbare Manschette besitzen, die mit Fluid befüllbar ist. Insbesondere kann das Blutdruckmessgerät 1 ein Handgelenksgerät sein, bei dem die Manschette passgenau um das Handgelenk eines Patienten gelegt werden kann. An der Manschette befestigt ist ein Gerätekorpus umfassend ein Gerätegehäuse, eine Pumpe 2 zur Fluidbefüllung und/oder -entleerung der Manschette, eine Energieversorgung 3 in Form von Batterien und/oder Akkus zum Betreiben der Pumpe 2, einen Messwertaufnehmer z. B. in Form eines Drucksensors 4, eine Steuereinrichtung 5 zur Ansteuerung der Pumpe 2 und zur Auswertung der Messsignale des Messwertaufnehmers 4 sowie eine Speichereinrichtung 6 zur Abspeicherung der Messwerte.

Wie Figur 1 zeigt, ist am Gerätekorpus des Blutdruckmessgeräts 1 eine Anschlussschnittstelle 7 vorgesehen, die mit der Speichereinrichtung 6 und/oder der Steuereinrichtung 5 verbunden ist. In der gezeichneten Ausführungsform bildet die Anschlussschnittstelle 7 einen RS-232-Port, wobei die Anschlussschnittstelle 7 erfindungsgemäß als Chipkartenkontaktlereinheit ausgebildet ist.

Mit der Anschlussschnittstelle 7 ist eine als separates Bauteil ausgebildete Datenübertragungseinheit 8 verbindbar, die einerseits eine interne Anschlussschnittstelle 9 aufweist, mittels derer sie an die RS-232-Schnittstelle 7 des Blutdruckmessgeräts angeschlossen werden kann. Andererseits umfasst die Datenübertragungseinheit 8 eine externe Datenübertragungsschnittstelle 10, die grundsätzlich verschiedene Formate aufweisen kann. Die externe Datenübertragungsschnittstelle 10 kann z. B. eine Infrarotübertragungsschnittstelle, ein Faxmodem, eine Blue Tooth-Einheit, ein Chipkartenterminal und/oder ein Anschlusskabelterminal sein.

Die Datenübertragungseinheit 8 besitzt vorteilhafterweise einen Schnittstellenwandler, der zwischen der internen Anschlussschnittstelle 9 und der externen Übertragungsschnittstelle 10 wirksam ist. Der Schnittstellenwandler transformiert das an der internen Anschlussschnittstelle 9 benötigte Daten- und Übertragungsformat, das durch den Anschluss an die RS-232-Schnittstelle 7 des Blutdruckmessgerätes bedingt ist, in das jeweils passende Daten- und Übertragungsformat, das an der externen Datenübertragungsschnittstelle 10 benötigt und durch die daran anzuschließende externe Datenverarbeitungsanlage bedingt wird. Der Schnittstellenwandler passt dabei nicht nur den Spannungspegel der einzelnen Datensignale an die Erfordernisse an, sondern transformiert auch das Datenformat und das Übertragungsprotokoll beispielsweise von RS-232-Format auf das von den Blue Tooth-Spezifikationen vorgeschriebene Format.

Vorteilhafterweise ist die Datenübertragungseinheit 8 mittels einer Steckverbindung mit dem Gerätekorpus des Blutdruckmessgerätes verbindbar. Insbesondere ist interne Anschlussschnittstelle 9 der Datenübertragungseinheit 8 ein Chipkartenadapter, der in die als Chipkartenkontaktiereinheit ausgebildete Anschlussschnittstelle 7 des Gerätekorpus des Blutdruckmessgeräts 1 einsteckbar ist. Hierdurch kann in einfacher Weise die Datenübertragungseinheit 8 ausgetauscht werden.

Wie Figur 2 zeigt, besitzt die Ausbildung der Anschlussschnittstelle 7 des Gerätekorpus des Blutdruckmessgerätes 1 in Form der Chipkartenkontaktiereinheft auch den Vorteil, dass unmittelbar an die Anschlussschnittstelle 7 des Gerätekorpus des Blutdruckmessgerätes 1 eine externe Datenverarbeitungsanlage, beispielsweise in Form eines Laptops oder eines PCs, angeschlossen werden kann. In der zuvor beschriebenen Weise kann hierdurch über die reine Datenübertragung hinaus die Auswertung der vom Blutdruckmessgerät bestimmten Messdaten und die Steuerung des Blutdruckmessgerätes am externen Datenverarbeitungsgerät 11 durchgeführt werden.

## Patentansprüche

1. Ein Blutdruckmessgerät (1), aufweisend:
eine Messvorrichtung (2, 3, 4, 5, 6) zur Bestimmung von Blutdruckdaten mit einer Gerätekorpus-Anschlussschnittstelle (7);
eine Datenübertragungseinheit (8), die einen separaten austauschbaren Schnittstellenwandler aufweist, welcher lösbar mit einem Gerätekorpus des Blutdruckmessgerätes (1) verbindbar ist und aufweist:
eine interne Anschlussschnittstelle (9), die an die Gerätekorpus-Anschlussschnittstelle (7) anschließbar ist;
eine externe Übertragungsschnittstelle (10) für:
Übertragung der Blutdruckdaten an externe Datenverarbeitungsgeräte (11) und/oder Übertragung von Steuerungsdaten an die Messvorrichtung (2, 3, 4, 5, 6);
wobei der Schnittstellenwandler ein zwischen seiner internen Anschlussschnittstelle (9) und seiner externen Übertragungsschnittstelle (10) zu der Messvorrichtung (2, 3, 4, 5, 6) passendes erstes Daten- und/oder Übertragungsformat in ein zu dem jeweiligen externen Datenverarbeitungsgerät passendes zweites Daten- und/oder Übertragungsformat wandelt,
**dadurch gekennzeichnet, dass**
die Gerätekorpus-Anschlussschnittstelle (7) eine Chipkartenkontaktiereinheit (12) mit Unterscheidungsmitteln aufweist, um zu ermitteln, ob eine mit Daten beschreibbare Chipkarte oder eine als Chipkartenadapter ausgebildete interne Anschlussschnittstelle (9) der Datenübertragungseinheit (8) in die Chipkartenkontaktiereinheit eingesetzt ist.

2. Das Blutdruckmessgerät nach Anspruch 1, wobei der Schnittstellenwandler einen Protokollwandler umfasst, betriebsfähig das Datenübertragungsprotokoll der internen Anschlussschnittstelle (9) mit dem davon verschiedenen Datenübertragungsprotokoll der externen Übertragungsschnittstelle (10) zu synchronisieren.

3. Das Blutdruckmessgerät nach einem der vorhergehenden Ansprüche, wobei der Schnittstellenwandler einen Pegelwandler umfasst, betriebsfähig den Datensignalpegel der internen Anschlussschnittstelle (9) an den davon verschiedenen Datensignalpegel der externen Übertragungsschnittstelle (10) anzupassen.

4. Das Blutdruckmessgerät nach einem der vorhergehenden Ansprüche, wobei die externe Übertragungsschnittstelle (10) wenigstens eines davon umfasst: eine Infrarotschnittstelle, ein Faxmodem, ein Chipkartenterminal, eine Kabelanschlussschnittstelle, eine Blue Tooth-Einheit, eine USB-Schnittstelle, einen Firewire-Port und eine WLAN-Schnittstelle.

5. Das Blutdruckmessgerät nach einem der vorhergehenden Ansprüche, wobei die Datenübertragungs-einrichtungseinheit (8) einen Akkulader betriebsfähig zur elektrischen Ladung eines im Gerätekorpus des Blutdruckmessgeräts (1) beinhalteten Akkus umfasst.

6. Das Blutdruckmessgerät nach einem der vorhergehenden Ansprüche, wobei die interne Anschlussschnittstelle (9) und die externe Anschlussschnittstelle (10) des Schnittstellenwandlers voneinander verschieden sind.

7. Das Blutdruckmessgerät nach einem der vorhergehenden Ansprüche, wobei die Datenübertragungseinheit (8) einen Aufsatz umfasst, betriebsfähig über eine Steckverbindung mit dem Gerätekorpus des Blutdruckmessgeräts verbunden zu sein.

8. Das Blutdruckmessgerät nach einem der vorhergehenden Ansprüche, wobei die Datenübertragungseinheit (8) bidirektional ist.

9. Das Blutdruckmessgerät nach einem der vorhergehenden Ansprüche, wobei das Blutdruckmessgerät als Handgelenksgerät ausgebildet ist.

## Claims

1. A sphygmomanometer (1), having:
a measuring device (2, 3, 4, 5, 6) for determining blood pressure data with an apparatus body connection interface (7);
a data transfer unit (8) which has a separate interchangeable interface converter which can be detachably connected to an apparatus body of the sphygmomanometer (1) and has:
an internal connection interface (9) which can be connected to the apparatus body connection interface (7);
an external transfer interface (10) for:
transfer of the blood pressure data to external data processing equipment (11) and/or transfer of control data to the measuring device (2, 3, 4, 5, 6) ;
wherein the interface converter converts a first data and/or transfer format passing between its internal connection interface (9) and its external transfer interface (10) to the measuring device (2, 3, 4, 5, 6) into a second data and/or transfer format passing to the relevant external data
processing apparatus,
**characterised in that**
the apparatus body connection interface (7) has a chip card contacting unit (12) with discrimination means to determine whether a data-writeable chip card or an internal connection interface (9), designed as a chip card adapter, of the data transfer unit (8) is inserted in the chip card contacting unit.

2. The sphygmomanometer according to claim 1, wherein the interface converter comprises a protocol converter to operably synchronise the data transfer protocol of the internal connection interface (9) with the data transfer protocol of the external transfer interface (10) which is different to it.

3. The sphygmomanometer according to one of the preceding claims, wherein the interface converter comprises a level converter to operably adapt the data signal level of the internal connection interface (9) to the data signal level of the external transfer interface (10) which is different to it.

4. The sphygmomanometer according to one of the preceding claims, wherein the external transfer interface (10) comprises at least one of: an infrared interface, a fax modem, a chip card terminal, a cable connection interface, a blue tooth unit, a USB interface, a firewire port and a WLAN interface.

5. The sphygmomanometer according to one of the preceding claims, wherein the data transfer unit (8) comprises a battery charger operable to electrically charge a battery contained in the apparatus body of the sphygmomanometer (1).

6. The sphygmomanometer according to one of the preceding claims, wherein the internal connection interface (9) and the external connection interface (10) of the interface converter are different from one another.

7. The sphygmomanometer according to one of the preceding claims, wherein the data transfer unit (8) comprises a cover to be operably connected via a plugin connection to the apparatus body of the sphygmomanometer.

8. The sphygmomanometer according to one of the preceding claims, wherein the data transfer unit (8) is bidirectional.

9. The sphygmomanometer according to one of the preceding claims, wherein the sphygmomanometer is designed as a wrist device.

## Revendications

1. Tensiomètre (1) comportant :
un dispositif de mesure (2, 3, 4, 5, 6) pour définir des données de tension artérielle, avec une interface de raccordement de corps d'appareil (7) ;
une unité de transmission de données (8) qui comporte un convertisseur d'interface échangeable séparé qui est apte à être relié de manière amovible à un corps d'appareil du tensiomètre (1) et qui comporte :
une interface de raccordement interne (9) qui est apte à être raccordée à l'interface de raccordement de corps d'appareil (7) ;
une interface de transmission externe (10) pour :
transmettre les données de tension artérielle à des appareils externes de traitement de données (11) et/ou transmettre des données de commande au dispositif de mesure (2, 3, 4, 5, 6) ;
étant précisé que le convertisseur d'interface convertit un premier format de données et/ou de transmission adapté, entre son interface de raccordement interne (9) et son interface de transmission externe (10), au dispositif de mesure (2, 3, 4, 5, 6), en un second format de données et/ou de transmission adapté à l'appareil externe de traitement de données,
**caractérisé en ce que** l'interface de raccordement de corps d'appareil (7) comporte une unité de contact pour carte à puce (12) avec des moyens de différenciation pour déterminer si une carte à puce apte à recevoir des données ou une interface de raccordement interne (9), conçue comme un adaptateur de carte à puce, de l'unité de transmission de données (8) est placée dans l'unité de contact pour carte à puce.

2. Tensiomètre selon la revendication 1, dans lequel le convertisseur d'interface comprend un convertisseur de protocole apte à synchroniser le protocole de transmission de données de l'interface de raccordement interne (9) avec le protocole de transmission de données, différent de celui-ci, de l'interface de transmission externe (10).

3. Tensiomètre selon l'une des revendications précédentes, dans lequel le convertisseur d'interface comprend un convertisseur de niveau apte à adapter le niveau de signaux de données de l'interface de raccordement interne (9) au niveau de signaux de données, différent de celui-ci, de l'interface de transmission externe (10).

4. Tensiomètre selon l'une des revendications précédentes, dans lequel l'interface de transmission externe (10) comprend l'un au moins des éléments suivants : une interface infrarouge, un modem télécopieur, un terminal de carte à puce, une interface de raccordement de câble, une unité Blue Tooth, une interface USB, un port Firewire et une interface WLAN.

5. Tensiomètre selon l'une des revendications précédentes, dans lequel l'unité de transmission de données (8) comprend un chargeur de batterie apte à charger électriquement une batterie contenue dans le corps d'appareil du tensiomètre (1).

6. Tensiomètre selon l'une des revendications précédentes, dans lequel l'interface de raccordement interne (9) et l'interface de raccordement externe (10) du convertisseur d'interface sont différentes.

7. Tensiomètre selon l'une des revendications précédentes, dans lequel l'unité de transmission de données (8) comprend une partie rapportée apte à être reliée par l'intermédiaire d'une connexion enfichable au corps d'appareil du tensiomètre.

8. Tensiomètre selon l'une des revendications précédentes, dans lequel l'unité de transmission de données (8) est bidirectionnelle.

9. Tensiomètre selon l'une des revendications précédentes, qui est conçu comme un appareil pour le poignet.
